# EUROPÄISCHE PATENTSCHRIFT

(11) **EP 2 520 225 B1**
(45) Veröffentlichungstag und Bekanntmachung des Hinweises auf die Patenterteilung: **21.05.2014**
(21) Anmeldenummer: 11003732.2
(22) Anmeldetag: 06.05.2011
(51) Int. Cl.: A61B 5/151

(54) **Lanzette**
Lancet
Lancette

(43) Veröffentlichungstag der Anmeldung: 07.11.2012
(73) Patentinhaber: Roche Diagnostics GmbH, 68305 Mannheim (DE); F.Hoffmann-La Roche AG, 4070 Basel (CH)
(72) Erfinder: Hoenes, Joachim, 64673 Zwingenberg (DE); List, Hans, 64754 Hesseneck-Kailbach (DE)
(74) Vertreter: Twelmeier Mommer & Partner

(56) Entgegenhaltungen:
- EP-A1- 1 360 932
- EP-A1- 1 911 394
- US-A- 3 046 987

## Beschreibung

Die Erfindung geht aus von einer Lanzette, die an einer Oberseite wenigstens einen nach oben offenen Kanal zur Probenaufnahme aufweist. Eine Lanzette mit den im Oberbegriff des Anspruchs 1 angegebenen Merkmalen ist aus der EP 1 360 932 A1 bekannt.

Beim Einstich in den Körper eines Patienten füllt sich der Kanal durch Kapillarkräfte mit Körperflüssigkeit, in der Regel Blut oder interstitieller Flüssigkeit. Lanzetten mit derartigen Kapillarkanälen ermöglichen deshalb eine einfache Entnahme von kleinen Körperflüssigkeitsproben und erleichtern somit Messungen der Konzentration von medizinisch bedeutsamen Analyten, beispielsweise Glucose oder Lactat.

Für eine Messung der Analytkonzentration muss die Probe zu einem Testfeld gebracht werden, das Nachweisreagenzien enthält. Ein solches Testfeld kann auf der Lanzette selbst angeordnet sein, wie dies aus der WO 2005/084530 A2 bekannt ist. Die Probe wird dann in dem Kanal durch Kapillarkräfte zu dem Testfeld transportiert. Eine Alternative zu Lanzetten mit integrierten Testfeldern sind Systeme, die aus Lanzetten und separaten Testelementen bestehen. Bei derartigen Systemen wird eine Probe aus dem Kanal auf das Testelement übertragen, indem die Lanzette gegen das Testelement gedrückt wird, d.h. die Oberseite der Lanzette bildet eine Anlagefläche zum Anlegen eines Testelements. Die in dem Kanal enthaltene Probe kann dann von dem Testelement aufgesaugt werden. Ein derartiges System ist beispielsweise aus der EP 2263526 A1 bekannt. Testelemente und Lanzetten können auf einem Trägerband angeordnet sein oder als lose, einzelne Teile des Systems vorliegen.

Das Anbringen von Testfeidern auf Lanzetten ist aus fertigungstechnischen Gründen aufwendig. Systeme, die aus Lanzetten und separaten Testelementen bestehen, haben deshalb den Vorteil einer vergleichsweise kostengünstigen Fertigung, allerdings ist die Übertragung von Probenflüssigkeit von einer Lanzette auf ein Testelement problematisch.

Aufgabe der vorliegenden Erfindung ist es, einen Weg aufzuzeigen, wie sich eine Flüssigkeitsprobe aus einem Kanal einer Lanzette besser auf ein separates Testelement übertragen lässt.

Diese Aufgabe wird durch eine Lanzette gelöst, die an einem vorderen Ende eine Lanzettenspitze hat und an einer Oberseite eine Anlagefläche zum Anlegen eines Testelements sowie wenigstens einen Kanal zur Probenaufnahme aufweist, wobei ein nach oben offener Abschnitt des Kanals in der Anlagefläche verläuft. Erfindungsgemäß hat der Kanal einen weiteren nach oben offenen Kanalabschnitt, der an der Oberseite der Lanzette in einer Fläche verläuft, die tiefer liegt als die Anlagefläche, und die Anlagefläche zwischen der Lanzettenspitze und der tiefer liegenden Fläche angeordnet ist.

Wenn die Oberseite einer erfindungsgemäßen Lanzette mit einem Testelement bedeckt wird, bildet sich zwischen der tiefer liegenden Fläche und dem Testelement ein Kapillarspalt. Durch Kapillarkräfte kann dann Probenflüssigkeit aus dem Kanal in den Kapillarspalt zwischen dem tiefer liegenden Oberflächenbereich der Lanzette und dem Testelement transportiert werden. Die der tiefer liegenden Fläche der Lanzette gegenüberliegende Oberfläche des Testelements kann auf diese Weise großflächig benetzt werden.

Während sich durch Bedecken des Kanals einer herkömmlichen Lanzette, der waagrecht in einer Ebene verläuft, nur eine Benetzung des Testelements entlang einer schmalen Linie, entlang welcher der Kanal das Testelement berührt, ergibt, lässt sich mit einer erfindungsgemäßen Lanzette ein verbesserter Probenübertrag mit einer spreitenden Wirkung kombinieren, so dass eine breitere Fläche auf dem Testelement benetzt wird. Ein Kapillarspalt zwischen der tiefer liegenden Fläche und einem zur Probenübergabe an die Anlagefläche angelegten Testelement füllt sich nämlich durch Kapillarkräfte sowohl in Richtung des Kanals als auch quer dazu. Insbesondere für eine fotometrische Auswertung einer Nachweisreaktion des Testfeldes ist dies ein bedeutsamer Vorteil. Wegen der spreitenden Wirkung bei einer Probenübertragung auf ein Testfeld wird die tiefer liegende Fläche der Lanzette auch als Spreitfläche bezeichnet.

Wenn der Abschnitt des Kapillarkanals in der Spreitfläche verläuft, bildet sich der Kapillarspalt auf beiden Seiten des Kanals. An sich genügt für eine spreitenden Probenübertragung aber, wenn sich der Kapillarspalt auf einer Seite des Kanals bildet. In diesem Fall kann sich die Anlagefläche auf einer Seite seitlich an dem Kanal entlang erstrecken, während auf der anderen Seite eines Kanalabschnitt die tiefer liegende Fläche ist, so dass sich nur auf dieser Seite beim Anlegen eines Testelements an die Anlagefläche der Lanzette ein Kapillarspalt bildet.

Indem der Kanal einer erfindungsgemäßen Lanzette in einen tiefer liegenden Oberflächenbereich hineinführt, wird erreicht, dass sich der Kanal nicht nur in einer einzigen Ebene erstreckt, sondern zusätzlich zu einem Flüssigkeitstransport in Längsrichtung des Kanals auch einen Transport in einer Richtung bewirkt, die eine Komponente senkrecht zur Oberseite der Lanzette hat, nämlich nach unten. Beim Bedecken des Kanals mit einem Testelement kann dann die Oberseite des Testelements einen Anfangsabschnitt des Kanals, d. h. einen vor der Vertiefung verlaufenden, oberen Kanalabschnitt, kontaktieren und dort von darin enthaltener Flüssigkeit benetzt werden.

Bei einer erfindungsgemäßen Lanzette wird unter 'vorne" das die Lanzettenspitze aufweisende Ende verstanden. Mit dem Wort 'hinten" wird dementsprechend das von der Lanzettenspitze abgewandte Ende bezeichnet. Die Angaben "vorn" und "hinten" beziehen sich also auf die Stichrichtung, nämlich die Richtung von der Lanzettenspitze entlang der Längsrichtung der Lanzette. Bei einer erfindungsgemäßen Lanzette wird mit der Oberseite die Seite der Lanzette bezeichnet, welche den Kanal zur Probenaufnahme und die Anlagefläche, auf die zur Probenübergabe ein Testelement gelegt wird, aufweist. Dementsprechend wird unter "oben" die Richtung senkrecht zur Stichrichtung und senkrecht zur Breite der Lanzette verstanden, in der Regel also die Richtung senkrecht zu der Anlagefläche. Somit bedeutet die Angabe "tiefer liegen" eine Position, die nach oben einen Abstand von einer geometrischen Ebene hat, in der die Anlagefläche liegt. Zu der Anlagefläche parallele Ebenen, die sich unterhalb von der Anlagefläche befinden, liegen also "tiefer".

Der Kapillarkanal kann in die tiefer liegende Fläche mit einem geneigten Kanalabschnitt hinunterführen. Möglich ist aber auch, dass der Kanal einen Grund hat, der auf gleich bleibender Höhe liegt, also der Kanal in der Anlagefläche tiefer ist als in der tiefer liegenden Fläche und dadurch der Höhenunterschied ausgeglichen wird.

Die tiefer liegende Fläche kann einen Boden einer nach oben offenen Vertiefung bilden, in die der Kanal hineinführt, insbesondere mit einem geneigten Kanalabschnitt. Indem die Oberseite der Lanzette eine Vertiefung aufweist, kann die Oberseite der Lanzette zwei Anlageflächen zum Anlegen an ein Testelement, beispielsweise einen Teststreifen, bilden, wobei die Vertiefung in Längsrichtung der Lanzette gesehen zwischen den beiden Anlageflächen liegt. Wird die Oberseite der Lanzette mit einem Testelement bedeckt wird, liegt dieses also an zwei Anlageflächen an, zwischen denen die tiefer liegende Fläche ist. Der sich zwischen Testelement und Lanzette bildende Kapillarspalt ist dann gut definiert, insbesondere wenn die beiden Anlageflächen auf gleicher Höhe liegen.

Allerdings lässt sich ein gut definierter Kapillarspalt zwischen Lanzette und Testelement auch mit einer Lanzette erreichen, die anstelle einer Vertiefung zwischen zwei Anlageflächen an ihrer Oberseite nur eine einzige Stufe aufweist, so dass als Anlagefläche oberhalb der tiefer liegenden Fläche nur eine einzige Fläche zur Verfügung steht. In einem solchen Fall kann anstelle eines ebenen Testelements ein Testelement verwendet werden, das einen Abstandshalter, beispielsweise eine Stufe aufweist. Als Abstandshalter kann beispielsweise auf einen Teil der Oberfläche eines Teststreifens ein Stück Folie geklebt werden, so dass sich eine Stufe bildet. Der Abstandhalter des Testelements wird für eine Probenaufnahme dann an die tiefer liegende Fläche der Lanzette angelegt, während an die Anlagefläche der Lanzette ein Abschnitt des Testelements vor dessen Abstandhalter angelegt wird. Ein solcher Abstandhalter kann an sich auch in ein Messgerät integriert sein, das ein Testelement an eine Lanzette anlegt und dann einen Analytgehalt in einer dadurch auf das Testgehalt übertragenen Probe misst, beispielsweise den Glucosegehalt.

Bei einem aus einer erfindungsgemäßen Lanzette und einem Testelement gebildeten System kann also ein Abstandshalter vorgesehen sein, der zur Ausbildung des Kapillarspalts einen Abstand zwischen einer Fläche an der Oberseite der Lanzette und einer Probenaufnahmefläche des Testelements vorgibt.

Eine erfindungsgemäße die Lanzette kann an ihrer Oberseite eine Vertiefung aufweisen, in die ein geneigter Abschnitt des Kanals hinunterführt und aus welcher ein weiterer geneigter Abschnitt des Kanals hinaufführt. Zwischen den beiden geneigten Abschnitten verläuft der Kanal in der Vertiefung, beispielsweise indem der Kanal in die Vertiefung eingeschnitten ist, etwa durch Ätzen oder Laserschneiden. Bei einem Stich in den Körper eines Patienten füllt sich der Kanal durch Kapillarkräfte mit Körperflüssigkeit. Die erfindungemäße Lanzette hat in diesem Fall also einen Kapillarkanal, der die nach oben offene Vertiefung durchquert, wobei ein Abschnitt des Kanals in einem Boden der Vertiefung angeordnet ist.

Ein geneigter Kanalabschnitt, der in die Vertiefung hinunter oder aus der Vertiefung hinauf führt, kann schräg ausgebildet sein, d.h. eine im wesentlichen konstante Steigung haben, oder gekrümmt sein, d.h. eine Steigung haben, die sich entlang des geneigten Abschnitts ändert.

Indem der Kanal die Vertiefung durchquert, lässt sich erreichen, dass sich ein Kapillarspalt zwischen einem Testelement, mit dem zur Übergabe einer Probe die Oberseite der Lanzette bedeckt wird, und der Oberfläche der Lanzette in der Vertiefung schnell füllt. Der Kanal ist bevorzugt in die Oberseite der Lanzette und in die Vertiefung eingeschnitten, beispielsweise durch Ätzen oder Laserschneiden.

Der Kanal kann als eine Rinne oder als ein Schlitz ausgebildet sein. Eine solche Rinne oder ein Schlitz kann bei einer erfindungsgemäßen Lanzette mit einem ersten Abschnitt zwischen der Spitze und der Vertiefung verlaufen, also in der Anlagefläche. Ein zweiter Abschnitt der Rinne oder des Schlitzes verläuft in einer Fläche, die tiefer liegt als die Anlagefläche, oder an einer solchen Fläche entlang.

Bevorzugt sind die Anlagefläche und die tiefer liegende Fläche jeweils eben. In der tiefer liegenden Fläche verläuft dann ein horizontaler Abschnitt des Kanals. Möglich ist es auch, dass an einen in die Vertiefung hinein führenden Abschnitt unmittelbar ein aus der Vertiefung hinaus führender Kanalabschnitt anschließt. Der in der Vertiefung verlaufende Abschnitt kann eben ausgebildet sein, also parallel zu einem in der Anlagefläche angeordneten verlaufenden Kanalabschnitt verlaufen.

Indem ein Kanalabschnitt vor der Vertiefung und ein weiterer Kanalabschnitt hinter der Vertiefung angeordnet sind, kann der Kanal eine größere Flüssigkeitsmenge aufnehmen, die dann zur Verfügung steht, um einen Kapillarspalt zwischen der Oberseite der Lanzette im Bereich der Vertiefung und dem Testelement zu füllen.

Eine weitere vorteilhafte Weiterbildung der Erfindung sieht vor, dass die Lanzette einen Schaft aufweist, der in der Lanzettenspitze endet, wobei die tiefer liegende Fläche zumindest teilweise in dem Schaft angeordnet ist. Unter einem Schaft wird ein schmaler Bereich einer Lanzette verstanden, der bei einem Stich ganz oder teilweise in den Körper eines Patienten eindringt. An den Schaft schließt bei vielen Lanzetten ein breiterer Abschnitt an, mit dem eine Lanzette beispielsweise in einer Halterung eines Stechgeräts gehalten werden kann.

An sich kann die Spreitfläche als tiefer liegende Fläche auch in einem an den Schaft anschließenden verbreiterten Abschnitt des Lanzettenkörpers angeordnet sein. Indem die Spreitfläche zumindest teilweise in dem Schaft angeordnet ist, lässt sich die bei einem Stich aufgenommene Probenflüssigkeit aber schneller in den Kanalabschnitt transportieren, der in oder an der tiefer liegenden Fläche, d.h. der Spreitfläche verläuft. Insbesondere kann ein Kanalabschnitt in der Spreitfläche sogar schon Probenflüssigkeit aufnehmen, während die Lanzette noch im Körper eines Patienten steckt. Bei einem Stich kann in einem solchen Fall der die Spreitfläche aufweisende Abschnitt des Schaftes vollständig oder teilweise in den Körper eines Patienten eindringen. Erforderlich ist dies jedoch nicht. Die Spreitfläche kann auf dem Schaft auch erst in einem so großen Abstand von der Spitze beginnen, dass die Spreitfläche bei einem Stich normalerweise nicht in den Körper des Patienten eindringt.

Eine weitere vorteilhafte Weiterbildung der Erfindung sieht vor, dass die Spreitfläche auf einer Seite oder auf beiden Seiten an Seitenflächen der Lanzette angrenzt. Dies bedeutet, dass sich die Spreitfläche bis in wenigstens eine Seitenfläche der Lanzette hinein erstreckt. Die Seitenflächen der Lanzette haben dann also Aussparungen oder Öffnungen. Im Bereich der Spreitfläche sind somit die seitlichen Ränder der Oberseite der Lanzette ebenfalls vertieft. Die Spreitfläche kann sich auf diese Weise über die volle Breite erstrecken, welche die Lanzette dort, also im Bereich der Spreitfläche, hat. Beim Anlegen eines Testelements wird so eine maximale Verteilung von Probenflüssigkeit auf dem Testelement quer zur Kanalrichtung ermöglicht. Ein Kapillarspalt zwischen einem auf die Oberseite der Lanzette gelegten Testelement und der Lanzette ist auf diese Weise nämlich quer zur Kanalrichtung nicht begrenzt, sondern kann sich über die volle Breite erstrecken, welche die Lanzette im Bereich der Spreitfläche hat. Insbesondere wenn die Spreitfläche ganz oder teilweise in dem Schaft der Lanzette angeordnet ist, lässt sich auf diese Weise eine vorteilhaft große Spreitung von Probenflüssigkeit auf einem Testelement erreichen.

Eine erfindungsgemäße Lanzette ist bevorzugt als eine Flachlanzette ausgebildet, also aus Blech oder einem anderen Bandmaterial hergestellt. Besonders bevorzugt ist die Lanzette aus Blech, wobei zwischen der tiefer liegende Fläche und der Anlagefläche, eine Höhendifferenz ist, die weniger als die Blechstärke, vorzugsweise weniger als die halbe Blechstärke, beträgt.

Eine weitere vorteilhafte Weiterbildung der Erfindung sieht vor, dass zwischen der Spreitfläche, also der tiefer liegenden Fläche, und der Anlagefläche eine Höhendifferenz ist, die kleiner als die maximale Tiefe des Kanals ist.

Bei einer erfindungsgemäßen Lanzette besteht zwischen einem Kanalabschnitt in der Spreitfläche und einem Kanalabschnitt in der Anlagefläche eine Höhendifferenz. Diese Höhendifferenz kann von einem geneigten Kanalabschnitt überwunden werden, der in die Vertiefung hinunter führt.

Unter der Höhendifferenz wird dabei der Unterschied zwischen den Höhen verstanden, auf denen sich in der Spreitfläche und in der Anlagefläche jeweils die oberen Ränder der betreffenden Kanalabschnitte, d. h. deren seitliche Begrenzungen, befinden. Die Tiefe eines Kanals ist bei einem als Rinne ausgebildeten Kanal vom Grund der Rinne bis zu ihrem oberen Rand zu messen. Bei einem als Schlitz ausgebildeten Kanal ist die Tiefe dagegen durch die Materialstärke des Lanzettenkörpers vorgegeben, in dem sich der Schlitz befindet.

Bevorzugt beträgt die Höhendifferenz weniger als die maximale Tiefe des Kanals. Ist die Lanzette aus Blech ausgeschnitten, beträgt die Höhendifferenz bevorzugt weniger als die Blechstärke, insbesondere weniger als die halbe Stärke des Blechs. Bevorzugt beträgt die Höhendifferenz mindestens 10 µm, besonders bevorzugt mindestens 20 µm, insbesondere mindestens 30 µm. Bevorzugt beträgt die Höhendifferenz weniger als 150 µm, besonders bevorzugt weniger als 100 µm, insbesondere weniger als 80 µm. Bevorzugt hat der Kanal in der Spreitfläche eine Breite, die zwischen der Hälfte und dem doppelten der Höhendifferenz beträgt. Besonders bevorzugt ist die Breite des Kanals in der Vertiefung größer als die Höhendifferenz.

An sich kann eine Lanzette mehrere Kanäle aufweisen, die beispielsweise nebeneinander angeordnet sein können, um die bei einem Stich aufgenommene Menge an Körperflüssigkeit zu erhöhen. Ein einziger Kanal ist aber ausreichend.

Die Spreitfläche kann zwischen zwei Biegezonen ausgebildet sein, in denen die Lanzette verbogen ist. In jeder dieser beiden Biegezonen verläuft dann jeweils ein geneigter Abschnitt des Kanals. Die Vertiefung kann beispielsweise durch Tiefziehen ausgebildet sein. Der Kanal kann beispielsweise geätzt sein.

Eine erfindungsgemäße Lanzette und ein Testelement, mit dem sich nach einem Lanzettenstich der Kanal der Lanzette bedecken lässt, bilden zusammen ein System. Bevorzugt ist dabei die Lanzette von einem Sterilschutz umgeben und das Testelement außerhalb des Sterilschutzes der Lanzette gelagert.

Die Erfindung betrifft auch ein Verfahren zum Übertragen einer Flüssigkeitsprobe aus einem als Rinne oder Schlitz ausgebildeten Kanal einer Lanzette auf ein Testelement, indem der Kanal nach einem Lanzettenstich mit dem Testelement bedeckt wird, wobei das Testelement an einen ersten Oberflächenbereich der Lanzette, in der ein erster Abschnitt des Kanals verläuft, angelegt wird, so dass sich zwischen einem zweiten Oberflächenbereich der Lanzette, in dem ein zweiter Abschnitt des Kanals verläuft, und dem Testelement ein Kapillarspalt bildet, in den durch Kapillarkraft Flüssigkeit aus dem Kanal eindringt. Der erste Oberflächenbereich der Lanzette bildet also eine Anlagefläche und der zweite Oberflächenbereich eine Spreitfläche.

Die Oberseite einer erfindungsgemäßen Lanzette bildet für ein solches Verfahren bevorzugt zwei Anlageflächen zum Anlegen an ein Testelement. Die Spreitfläche liegt dann in Längsrichtung der Lanzette gesehen zwischen den beiden Anlageflächen, die bevorzugt eben sind und auf gleicher Höhe liegen.

Weitere Einzelheiten und Vorteile der Erfindung werden an einem Ausführungsbeispiel unter Bezugnahme auf die beigefügten Zeichnungen erläutert. Es zeigen:
- Figur 1: ein Ausführungsbeispiel einer Lanzette, und
- Figur 2: eine schematische Darstellung eines vorderen Abschnitts der Lanzette mit einem zur Probenübertragung an die Lanzette angelegten Testelement.

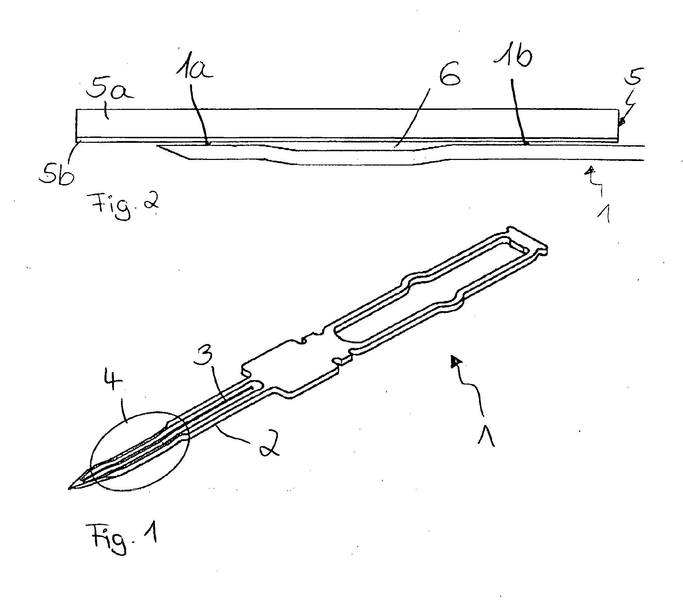

Die in Figur 1 mit Blick auf ihre Oberseite dargestellte Lanzette 1 hat einen Lanzettenkörper mit einem Schaft 2, der in einer Spitze endet. Die Lanzette 1 hat auf ihrer Oberseite einen nach oben offenen Kanal 3 zur Probenaufnahme. Der Kanal 3 ist bei dem dargestellten Ausführungsbeispiel als eine Rinne ausgebildet, kann aber beispielsweise auch ein Schlitz sein, der von der Oberseite bis zur Unterseite der Lanzette 1 durchgeht. Wenn mit der Lanzette 1 in den Körper eines Patienten eingestochen wird, füllt sich der Kanal 3 durch Kapillarkräfte mit Körperflüssigkeit. Der Kanal 3 ist also ein Kapillarkanal.

In dem eingekreisten Bereich 4 hat die Oberseite der Lanzette 1 eine nach oben offene Vertiefung, d.h. die Vertiefung ist unbedeckt. In die Vertiefung führt der Kanal 3 mit einem geneigten Abschnitt hinunter. Der Kanal 3 hat also einen abfallenden Abschnitt, der einen Höhenunterschied zwischen dem Boden der Vertiefung und der Oberseite der Lanzette 1 neben der Vertiefung, überwindet.

Um eine Körperflüssigkeitsprobe aus dem Kanal 3 auf ein Testelement 5 zu übertragen, wird die Lanzette 1 mit ihrer Oberseite an ein Testelement 5 angelegt, wie dies schematisch in Figur 2 dargestellt ist. Die Oberseite der Lanzette 1 weist also Anlageflächen zum Anlegen an ein Testelement 5 auf, wobei sich die Vertiefung zwischen den beiden in Figur 2 schematisch angedeuteten Anlageflächen 1 a, 1b befindet. Beim Anlegen eines Testelements 5 an die Lanzette 1 bildet sich im Bereich der Vertiefung zwischen dem Testelement 5 und der Lanzette 1 ein Kapillarspalt 6. Der Kapillarspalt 6 füllt sich mit Probenflüssigkeit aus dem Kanal 3 und bewirkt somit eine Spreitung beim Probenübertrag. Der Boden der Vertiefung wird deshalb als Spreitfläche bezeichnet.

Der Kanal 3 beginnt mit einem Anfangsabschnitt in der vorderen Anlagefläche 1a, die zwischen der Spitze und der Vertiefung angeordnet ist. Der Kanal 3 erstreckt sich durch die Vertiefung hindurch, wie dies in Figur 1 dargestellt ist. Ein Abschnitt des Kanals 3 ist also im Boden der Vertiefung angeordnet. Von der Lanzettenspitze aus gesehen führt der Kanal zunächst in die Vertiefung hinunter und wieder aus der Vertiefung hinauf. Mit anderen Worten hat der Kanal 3 also zwei geneigte Abschnitte. In einem geneigten Kanalabschnitt haben auch die Kanalränder einen abfallenden Verlauf.

Bevorzugt sind ein in die Vertiefung hinunter und ein aus der Vertiefung hinaufführender Abschnitt des Kanals 3 durch einen ebenen Kanalabschnitt miteinander verbunden. Der ebene Kanalabschnitt kann kürzer als der in die Vertiefung hinabführende Kanalabschnitt sein, ist bevorzugt aber länger, beispielsweise wenigstens doppelt so lang.

Bei dem dargestellten Ausführungsbeispiel ist die Vertiefung zwischen zwei eben verlaufenden Abschnitten des Kanals 3 angeordnet. Ein erster ebener Abschnitt des Kanals 3 ist von der Spitze der Lanzette 1 ausgesehen vor der Vertiefung angeordnet. Ein zweiter eben verlaufender Abschnitt des Kanals 3 ist von der Spitze ausgesehen hinter der Vertiefung angeordnet. Bei dem dargestellten Ausführungsbeispiel beginnt der Kanal 3 erst in einem kurzen Abstand von der Spitze, d.h. in einem Abstand von dem in Stichrichtung vorderen Ende der Lanzette 1. Der Kanal 3 kann aber auch bis zur Spitze hin durchgehen, also in Stichrichtung offen sein.

Wenn die Lanzette 1 mit der Oberseite an ein Testelement 5 angelegt wird, wird das Testelement 5 von den beiden Kanalabschnitten, zwischen denen sich die Vertiefung befindet, benetzt. Der zwischen der Lanzette 1 im Bereich der Vertiefung und dem Testelement 5 gebildete Kapillarspalt 6 füllt sich dann von beiden Enden aus.

Der Kanal 3 kann die Oberfläche der Lanzette 1 und insbesondere in die Vertiefung beispielsweise durch Ätzen eingeschnitten werden. Wie Figur 2 zeigt, kann sich die Vertiefung bis in die Seitenflächen der Lanzette 1 hineinerstrecken. Die Vertiefung erstreckt sich dann über die gesamte Breite der Lanzette 1 an dieser Stelle.

Als Material für die Lanzette 1 wird bevorzugt Blech verwendet. Prinzipiell kann die Lanzette aber auch aus anderen Materialien hergestellt werden, beispielsweise Kunststoff oder Keramik. Flachlanzetten lassen sich kostengünstig herstellen, indem ein Lanzettenkörper aus einem Bandmaterial, beispielsweise Blech, ausgeschnitten wird, beispielsweise durch Ätzen, Stanzen oder Laserschneiden.

Die Tiefe der Vertiefung gibt die Breite des in Figur 2 dargestellten Kapillarspalts 6 zwischen Testelement 5 und Lanzette 1 vor. Für eine gute Kapillarwirkung genügt bereits eine Höhendifferenz von 10 µm zwischen den an dem Testelement 5 anliegenden Bereichen der Lanzette 1 und der Vertiefung. Beispielsweise kann die Vertiefung zwischen 10 µm und 200 µm tief sein. Die Tiefe der Vertiefung stimmt bevorzugt mit der Höhendifferenz zwischen einem Kanalabschnitt in der Vertiefung und einem Kanalabschnitt außerhalb der Vertiefung überein.

Bevorzugt beträgt die Tiefe der Vertiefung weniger als die Materialstärke des Lanzettenkörpers, also beispielsweise weniger als die Stärke des Blechs, aus dem der Lanzettenkörper ausgeschnitten sein kann. Beispielsweise kann die Tiefe der Vertiefung weniger als die Hälfte der Materialstärke betragen.

Da bereits eine kleine Vertiefung ausreichend ist, kann die Tiefe der Vertiefung kleiner als die maximale Tiefe des Kanals 3 sein. Die Tiefe des Kanals 3 kann in der Vertiefung im Wesentlichen konstant sein und insbesondere mit der Kanaltiefe eines Kanalabschnitts außerhalb der Vertiefung übereinstimmen. Bei dem dargestellten Ausführungsbeispiel beträgt die Tiefe der Vertiefung zwischen 10% und 80% der Tiefe des Kanals 3 in der Vertiefung. Eine erfindungsgemäße Lanzette lässt sich aber auch mit abweichenden Maßen der Vertiefung herstellen als hier im Rahmen der Figurenbeschreibung genannt sind.

Bei dem dargestellten Ausführungsbeispiel hat der Kanal 3 in der Vertiefung eine Breite, die zwischen der Hälfte und dem Doppelten der Höhendifferenz beträgt, beispielsweise hat der Kanal 3 in der Vertiefung eine Breite, die größer als die Höhendifferenz ist. Zwingend erforderlich sind derartige Maße aber nicht. Der Kanal 3 kann in der Vertiefung eine im Wesentlichen konstante Breite haben, wie dies in Figur 1 dargestellt ist.

Der Kanal 3 kann in der Vertiefung eine im Wesentlichen konstante Breite haben. Insbesondere kann der Kanal 3 in der Vertiefung dieselbe Breite wie in Abschnitten vor und hinter der Vertiefung haben. Der Kanal 3 kann über seine gesamte Länge im Wesentlichen geradlinig verlaufen, wie dies in Figur 1 dargestellt ist. Ein solcher Kanal 3 lässt sich insbesondere auf einem schmalen Lanzettenschaft 2 gut anordnen. An sich ist es aber auch möglich, dass der Kanal 3 Kurven aufweist.

Bei dem dargestellten Ausführungsbeispiel ist die Vertiefung vollständig in dem Lanzettenschaft 2 angeordnet. An sich kann die Vertiefung aber auch in einem an den Schaft 2 anschließenden breiteren Abschnitt des Lanzettenkörpers angeordnet sein. Indem die Vertiefung aber zumindest teilweise in dem Schaft 2 angeordnet ist, lässt sich erreichen, dass sich der Kanal 3 im Bereich der Vertiefung bei einem Stich schneller füllt und eine Probe somit schneller auf zuverlässiger auf die Oberfläche der Lanzette 1 aufgelegten Testelement 5 übertragen werden kann.

Das in Figur 2 schematisch dargestellte Testelement 5 bildet zusammen mit der Lanzette 1 ein System. Das Testelement 5 hat einen Träger 5a mit einer Schicht aus Nachweisreagenzien 5b, die bei Anwesenheit einer Flüssigkeitsprobe eine Nachweisreaktion bewirken, beispielsweise zur fotometrischen Konzentrationsbestimmung. Die Lanzette 1 und das Testelement 5 sind so ausgebildet, dass sich beim Bedecken des Kanals 3 der Lanzette 1 mit dem Testelement 5 zwischen dem Testelement 5 und der Lanzette 1 im Bereich der Vertiefung ein Kapillarspalt 6 bildet, der Flüssigkeit aus dem Kanal 3 saugt. Bei dem dargestellten Ausführungsbeispiel hat das Testelement 5 eine plane Oberfläche, die an planen Oberflächenabschnitten der Lanzette 1, zwischen denen sich die Vertiefung befindet, an zu legen ist. Prinzipiell ist es aber auch möglich, das Testelement 5 mit einer gebogenen Oberfläche auszubilden und die Oberseite der Lanzette 1 entsprechend passend dazu zu formen.

Das Testelement 5 und die Lanzette 1 können in unterschiedlichen Magazinen verpackt sein oder in einem gemeinsamen Magazin gelagert werden. Ein Testelement wird aber jedenfalls erst nach einem Lanzettenstich an die Anlagefläche 1 a, 1 b der Lanzette 1 angelegt. Bis zu einem Lanzettenstich ist die Lanzette von einem Sterilschutz umgeben, der Beispielsweise als eine Magazinkammer oder eine Schutzfolie, die zusammen mit einem Trägerband ein die Lanzette enthaltendes Volumen umschließt, ausgebildet sein kann. Auf einem solchen Trägerband können mehrere Lanzetten angeordnet sein. Zwischen den Lanzetten können auf dem Trägerband Testelemente angeordnet sein.

### Bezugszahlen

- 1: Lanzette
- 1a, b: Anlagefläche
- 2: Schaft
- 3: Kanal
- 4: Bereich
- 5: Testelement
- 6: Kapillarspalt

## Patentansprüche

1. Lanzette, die an einem vorderen Ende eine Lanzettenspitze hat und an einer Oberseite eine Anlagefläche (1 a) zum Anlegen eines Testelements (5) sowie wenigstens einen Kanal (3) zur Probenaufnahme aufweist, **dadurch gekennzeichnet, dass** ein nach oben offener Abschnitt des Kanals (3) in der Anlagefläche (1 a) verläuft, und
der Kanal (3) einen weiteren nach oben offenen Kanalabschnitt aufweist, der an der Oberseite der Lanzette in einer Fläche verläuft, die tiefer liegt als die Anlagefläche (1 a), wobei die Anlagefläche (1 a) zwischen der Lanzettenspitze und der tiefer liegenden Fläche angeordnet ist, so dass sich zwischen der tieferliegenden Fläche und dem Testelement (5) ein Kapillarspalt (6) bildet, wenn die Oberseite der Lanzette mit dem Testelement (5) bedeckt wird.

2. Lanzette nach Anspruch 1, **dadurch gekennzeichnet, dass** die beiden Kanalabschnitte durch einen geneigten Kanalabschnitt verbunden sind, der in die tiefer liegende Fläche hinunter führt.

3. Lanzette nach einem der vorstehenden Ansprüche, **dadurch gekennzeichnet, dass** die Lanzette (1) einen Schaft (2) aufweist, wobei die tiefer liegende Fläche zumindest teilweise in dem Schaft (2) angeordnet ist.

4. Lanzette nach einem der vorstehenden Ansprüche, **dadurch gekennzeichnet, dass** die Lanzette aus Blech ist, wobei zwischen der tiefer liegende Fläche und der Anlagefläche (1a), eine Höhendifferenz ist, die weniger als die Blechstärke, vorzugsweise weniger als die halbe Blechstärke, beträgt.

5. Lanzette nach einem der vorstehenden Ansprüche, **dadurch gekennzeichnet, dass** zwischen der tiefer liegenden Fläche und der Anlagefläche (1 a) eine Höhendifferenz ist, die kleiner als die maximale Tiefe des Kanals (3) ist.

6. Lanzette nach einem der vorstehenden Ansprüche, **dadurch gekennzeichnet, dass** der in der Anlagefläche (1a) verlaufende Abschnitt des Kanals (3) ein Anfangsabschnitt des Kanals (3) ist.

7. Lanzette nach einem der vorstehenden Ansprüche, **dadurch gekennzeichnet, dass** die tiefe liegende Fläche einen Boden einer Vertiefung bildet, in die der Kanal (3) hineinführt.

8. Lanzette nach Anspruch 7, **dadurch gekennzeichnet, dass** der Kanal (3) die Vertiefung durchquert.

9. Lanzette nach Anspruch 7 oder 8, **dadurch gekennzeichnet, dass** die Oberseite eine weitere Anlagefläche (1 b) zum Anlegen an ein Testelement (5) aufweist, wobei die Vertiefung in Längsrichtung der Lanzette gesehen zwischen den beiden Anlageflächen (1a, 1b) liegt, und wobei die Anlageflächen (1a, 1b) eben sind und auf gleicher Höhe liegen.

10. Lanzette nach einem der vorstehenden Ansprüche, **dadurch gekennzeichnet, dass** die tiefer liegende Fläche an Seitenflächen der Lanzette (1) angrenzt.

11. System mit einer Lanzette nach einem der vorstehenden Ansprüche und einem Testelement (5), mit dem sich nach einem Lanzettenstich der Kanal (3) der Lanzette (1) bedecken lässt, wobei das Testelement (5) einen Träger mit einer Schicht aus Nachweisreagenzien aufweist, die bei Anwesenheit einer Flüssigkeitsprobe eine Nachweisreaktion bewirken, wobei die Lanzette (1) und das Testelement (5) so ausgebildet sind, dass sich durch Bedecken des Kanals (3) der Lanzette (1) mit dem Testelement (5) zwischen dem Testelement (5) und der Lanzette (1) ein Kapillarspalt bildet, der Flüssigkeit aus dem Kanal (3) saugt.

12. System nach Anspruch 11, **dadurch gekennzeichnet, dass** sich der Kapillarspalt zwischen dem Testelement (5) und einer Fläche an der Oberseite der Lanzette (1) bildet, die tiefer als eine Anlagefläche der Lanzette (1) liegt und in oder an der entlang ein nach oben offener Abschnitt des Kanals (3) verläuft.

13. System nach Anspruch 11 oder 12, **dadurch gekennzeichnet, dass** sich der Kapillarspalt zwischen dem Testelement (5) und einer Vertiefung, die an der Oberseite der Lanzette (1) vorgesehen ist und in die der nach oben offene Kanal (3) hineinführt, bildet.

14. System nach einem der Ansprüche 11 bis 13, **dadurch gekennzeichnet, dass** ein Abstandshalter vorgesehen ist, der zur Ausbildung des Kapillarspalts einen Abstand zwischen einer Fläche an der Oberseite der Lanzette (1) und einer Probenaufnahmefläche des Testelements (5) vorgibt.

15. Verfahren zum Übertragen einer Flüssigkeitsprobe aus einem als Rinne oder Schlitz ausgebildeten Kanal (3) einer Lanzette (1) auf ein Testelement (5) zur Untersuchung der Flüssigkeitsprobe, indem der Kanal (3) nach einem Lanzettenstich mit dem Testelement (5) bedeckt wird, **dadurch gekennzeichnet, dass** an einen Oberflächenbereich der Lanzette (1), in dem ein Anfangsabschnitt des Kanals (3) verläuft, das Testelement (5) angelegt wird, so dass sich zwischen einem zweiten Oberflächenbereich der Lanzette (1), in dem ein weiterer Abschnitt des Kanals (3) verläuft, und dem Testelement (5) ein Kapillarspalt (6) bildet, in den durch Kapillarkraft Flüssigkeit aus dem Kanal (3) eindringt.

## Claims

1. Lancet comprising
a lancet tip at a front end, and
an upper face with a contact surface (1a) for placing a test element (5) thereon, said upper face comprising at least one channel (3) for receiving a sample, **characterized in that**
an upwardly open section of the channel (3) running in the contact surface (1 a), and
the channel (3) has another upwardly open channel section, which runs on the upper face of the lancet in a surface area that is lower than the contact surface (1a), wherein the contact surface (1a) is arranged between the lancet tip and the lower surface area so that a capillary gap (6) forms between the lower surface area and a test element (5) when the upper face of the lancet is covered with the test element (5).

2. Lancet according to claim 1, **characterized in that** the two channel sections are connected by an inclined channel section which leads downward into the lower surface area.

3. Lancet according to any one of the preceding claims, **characterized in that** the lancet (1) comprises a shaft (2), wherein the lower surface area is arranged at least partially in the shaft (2).

4. Lancet according to any one of the preceding claims, **characterized in that** the lancet is made of sheet metal, wherein a difference in height exists between the lower surface area and the contact surface (1a), the difference being less than the metal sheet thickness, and more preferably less than half the metal sheet thickness.

5. Lancet according to any one of the preceding claims, **characterized in that** there is a difference in height between the lower surface area and the contact surface (1a), said difference being smaller than the maximum depth of the channel (3).

6. Lancet according to any one of the preceding claims, **characterized in that** the section of the channel (3) in the contact surface (1a) is a starting section of the channel (3).

7. Lancet according to any one of the preceding claims, **characterized in that** the lower surface area forms a base of a depression into which the channel (3) leads.

8. Lancet according to claim 7, **characterized in that** the channel (3) traverses the depression.

9. Lancet according to claim 7 or 8, **characterized in that** the upper face comprises a further contact surface (1b) for placing a test element (5) thereon, wherein the depression is located between the two contact surfaces (1a, 1b), as seen looking in the longitudinal direction of the lancet, and wherein the contact surfaces (1a, 1b) are flat and located at the same height.

10. Lancet according to any one of the preceding claims, **characterized in that** the lower surface area adjoins lateral surfaces of the lancet (1).

11. System comprising a lancet according to any one of the preceding claims and a test element (5), by which the channel (3) of the lancet (1) can be covered after lancing, wherein the test element (5) comprises a carrier having a layer of detection reagents which effect a detection reaction in the presence of a liquid sample, wherein the lancet (1) and the test element (5) are designed such that by covering the channel (3) of the lancet (1) with the test element (5) a capillary gap forms between the test element (5) and the lancet (1), the capillary gap taking up liquid from the channel (3).

12. System according to claim 11, **characterized in that** the capillary gap forms between the test element (5) and a surface area on the upper face of the lancet (1) which is lower than a contact surface of the lancet (1) and in or along which an upwardly open section of the channel (3) runs.

13. System according to claim 11 or 12, **characterized in that** the capillary gap forms between the test element (5) and a depression which is provided on the upper face of the lancet (1) and into which the upwardly open channel (3) leads.

14. System according to any one of claims 11 to 13, **characterized in that** a spacer is provided, which predetermines a distance between a surface area on the upper face of the lancet (1) and a sample receiving surface area of the test element (5) so as to form the capillary gap.

15. Method for transferring a liquid sample from a channel (3) of a lancet (1) which is designed as a groove or a slot to a test element (5) for analysis of the liquid sample by covering the channel (3) with the test element (5), **characterized in that** the test element (5) is placed on a surface region of the lancet (1) in which a starting section of the channel (3) runs, whereby a capillary gap (6) forms between a second surface region of the lancet (1) in which a second section of the channel (3) runs and the test element (5), with liquid from the channel (3) penetrating into the capillary gap due to capillary forces.

## Revendications

1. Lancette ayant à son extrémité avant une pointe de lancette et sur un côté supérieur une surface d'application (1a) pour appliquer un élément de test (5), et comportant au moins un canal (3) pour la réception d'un échantillon, **caractérisée en ce qu'**un tronçon du canal (3), ouvert vers le haut, s'étend dans la surface d'application (1a), et
le canal (3) comporte un autre tronçon de canal ouvert vers le haut qui s'étend, sur le côté supérieur de la lancette, dans une surface située plus bas que la surface d'application (1a), la surface d'application (1a) étant placée entre la pointe de lancette et ladite surface située plus bas de telle sorte qu'il se forme entre ladite surface située plus bas et l'élément de test (5) une fente capillaire (6) lorsque l'élément de test (5) vient recouvrir le côté supérieur de la lancette.

2. Lancette selon la revendication 1, **caractérisée en ce que** les deux tronçons de canal sont reliés par un tronçon de canal incliné qui descend dans la surface située plus bas.

3. Lancette selon l'une des revendications précédentes, **caractérisée en ce que** la lancette (1) comprend une tige (2), la surface située plus bas étant positionnée au moins partiellement dans la tige (2).

4. Lancette selon l'une des revendications précédentes, **caractérisée en ce que** la lancette est en tôle, une différence de hauteur, prévue entre la surface située plus bas et la surface d'application (1a), étant inférieure à l'épaisseur de la tôle, de préférence inférieure à la moitié de l'épaisseur de la tôle.

5. Lancette selon l'une des revendications précédentes, **caractérisée en ce qu'**une différence de hauteur, prévue entre la surface située plus bas et la surface d'application (1a), est inférieure à la profondeur maximale du canal (3).

6. Lancette selon l'une des revendications précédentes, **caractérisée en ce que** le tronçon du canal (3), qui s'étend dans la surface d'application (1a), est un tronçon initial du canal (3).

7. Lancette selon l'une des revendications précédentes, **caractérisée en ce que** la surface située plus bas forme le fond d'une cavité dans laquelle mène le canal (3).

8. Lancette selon la revendication 7, **caractérisée en ce que** le canal (3) traverse la cavité.

9. Lancette selon la revendication 7 ou 8, **caractérisée en ce que** le côté supérieur comprend une autre surface d'application (1 b) destinée à s'appliquer contre un élément de test (5), la cavité, vue dans le sens longitudinal de la lancette, se trouvant entre les deux surfaces d'application (1a, 1b) et lesdites surfaces d'application (1a, 1b) étant planes et se trouvant à la même hauteur.

10. Lancette selon l'une des revendications précédentes, **caractérisée en ce que** la surface située plus bas est adjacente à des faces latérales de la lancette (1).

11. Système comprenant une lancette selon l'une des revendications précédentes et un élément de test (5) apte à recouvrir le canal (3) de la lancette (1) après une piqûre de la lancette, l'élément de test (5) comprenant un support pourvu d'une couche de réactifs de détection, lesquels engendrent une réaction de détection en présence d'un échantillon de liquide, la lancette (1) et l'élément de test (5) étant réalisés de telle sorte que lorsque l'élément de test (5) vient recouvrir le canal (3) de la lancette (1), il se forme entre l'élément de test (5) et la lancette (1) une fente capillaire qui aspire du liquide hors du canal (3).

12. Système selon la revendication 11, **caractérisé en ce que** la fente capillaire se forme entre l'élément de test (5) et une surface située sur le côté supérieur de la lancette (1), surface qui est placée plus bas qu'une surface d'application de la lancette (1) et dans laquelle, ou le long de laquelle s'étend un tronçon ouvert vers le haut du canal (3).

13. Système selon la revendication 11 ou 12, **caractérisé en ce que** la fente capillaire se forme entre l'élément de test (5) et une cavité prévue sur le côté supérieur de la lancette (1) et dans laquelle mène le canal (3) ouvert vers le haut.

14. Système selon l'une des revendications 11 à 13, **caractérisé en ce qu'**il est prévu une entretoise qui, pour la formation de la fente capillaire, impose un écartement entre une surface située sur le côté supérieur de la lancette (1) et une surface de réception d'échantillon de l'élément de test (5).

15. Procédé pour transférer un échantillon de liquide d'un canal (3) se présentant sous forme de rainure ou de fente, d'une lancette (1) sur un élément de test (5) pour examiner l'échantillon de liquide en recouvrant le canal (3) avec l'élément de test (5) après une piqûre de la lancette, **caractérisé en ce que** l'on applique l'élément de test (5) contre une région de surface de la lancette (1) dans laquelle s'étend un tronçon initial du canal (3), de telle sorte qu'il se forme entre une seconde région de surface de la lancette (1) où s'étend un autre tronçon du canal (3) et l'élément de test (5) une fente capillaire (6) dans laquelle pénètre par capillarité du liquide provenant du canal (3).
